# EUROPEAN PATENT SPECIFICATION

(11) **EP 2 700 449 B1**
(45) Date of publication and mention of the grant of the patent: **15.07.2015**
(21) Application number: 13181256.2
(22) Date of filing: 21.08.2013
(51) Int. Cl.: B01L 3/00, G01N 33/558

(54) **Immunochromatographic Device**
Immunochromatographische Vorrichtung
Dispositif immunochromatographique

(30) Priority: 22.08.2012 JP 2012183560
(43) Date of publication of application: 26.02.2014
(73) Proprietor: Morinaga & Co., Ltd., Tokyo 108-8403 (JP)
(72) Inventor: Honjo, Tsutomu, Yokohama (JP)
(74) Representative: Creek, Isobel Clare

(56) References cited:
- WO-A1-93/13856
- CN-A- 1 464 304
- US-A1- 2008 204 745

## Description

### Technical field

The present invention relates to an immunochromatographic device.

### Background Art

Immunochromatographic assay is widely applied to kits for detecting antigens such as influenza viruses (see, for example, JP-A-2011-257138 corresponding to US2012028246 A1). In the kits, nitrocellulose or glass fiber filter, for example, is used as a test strip (see, for example, JP-A-2011-257138, supra).

WO93/13856 describes an assay apparatus having a first assay zone on a first solid support for capturing a species to be assayed. The apparatus also has a second assay zone on a second solid support component having an assay reagent thereon. The zones are slidable with respect to one another such that they can be brought into mutual contact.

The present invention is directed to provide an immunochromatographic device with a high quantitative ability.

### Summary of the invention

One embodiment of the present invention is an immunochromatographic device comprising: a glass plate including: a sample application portion for applying a sample containing an antigen to the device; a sample recovery portion for recovering the sample applied to the sample application portion from the device; a developing portion for developing the sample from the sample application portion to the sample recovery portion; and an antibody-carrying portion for carrying an antibody capable of binding to the antigen in the developing portion; wherein the developing portion comprises a transparent plate, the glass plate and the transparent plate are placed in parallel with each other with a gap, and the sample can migrate in the gap of the developing portion by capillary action; wherein the device is characterised in that the developing portion (13) or the antibody-carrying portion (14) in the glass plate (15) comprises a roughened surface, and the roughened surface has a mean roughness of 0.01-20.0 µm, a maximum height of roughness of 0.1-150.0 µm, and a concave-to-convex distance of 10-2000 µm.
It is preferable that the gap has a size of 3-50 µm.

It is preferable that the transparent plate has a portion comprising a roughened surface at an area corresponding to the roughened surface(s) of the developing portion and/or the antibody-carrying portion of the glass plate. In addition, it is preferable that each of the developing portion and the antibody-carrying portion of the glass plate and the transparent plate have a roughened surface, and does not comprise a spacer for keeping the gap.

Another embodiment of the present invention is a method for detecting an antigen in a sample using an immunochromatographic device, the device comprising a glass plate, the glass plate comprising a sample application portion for applying the sample to the device; a sample recovery portion for recovering the sample applied to the sample application portion from the device; a developing portion for developing the sample from the sample application portion to the sample recovery portion; and an antibody-carrying portion for carrying an antibody capable of binding to the antigen in the developing portion; wherein the developing portion comprises a transparent plate, the transparent plate is placed in parallel with the glass plate with a gap, and the sample can migrate in the gap of the developing portion by capillary action; the method comprising the steps of: applying the sample to the sample application portion to develop the sample through the developing portion; recovering the sample developed through the developing portion at the sample recovery portion; and detecting the antigen bound to the antibody-carrying portion; wherein the developing portion or the antibody-carrying portion comprises a roughened surface and the roughened surface has a mean roughness of 0.01-20.0 µm, a maximum height of roughness of 0.1-150.0 µm, and a concave-to-convex distance of 10-2000 µm.

### Brief Description of the Drawings

Fig. 1 is a diagrammatic representation of an immunochromatographic device according to one embodiment of the present invention;
Fig. 2 is a diagrammatic representation of some components of the immunochromatographic device according to one embodiment of the present invention;
Fig. 3 is a view illustrating a general procedure of immunochromatographic assay performed using the immunochromatographic device according to one embodiment of the present invention;
Fig. 4 is a diagrammatic representation of an immunochromatographic device according to another embodiment of the present invention; and
Fig. 5 is a diagrammatic representation of an immunochromatographic device with two glass plates put together directly without any spacer in one embodiment of the present invention.

### Preferred embodiments for carrying out the invention

The above and further objects, features, advantages, and ideas of the present invention are apparent from those skilled in the art from consideration of the detailed description of this specification. Furthermore, those skilled in the art can easily reproduce the present invention from these descriptions. The mode(s) and specific example(s) described below represent a preferable embodiment of the present invention, which is given for the purpose of illustration or description. The present invention is limited only by the scope of the appended claims.

### == Immunochromatographic device ==

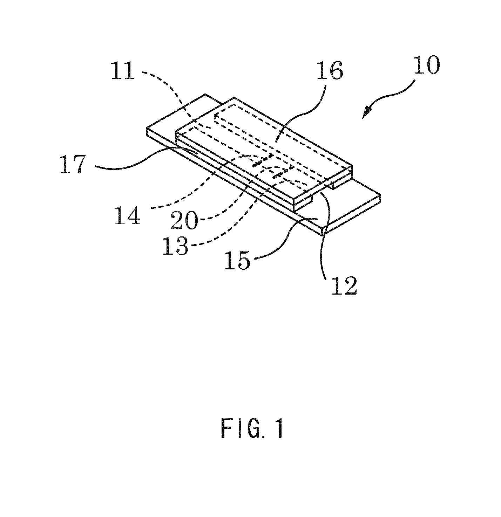

An immunochromatographic device 10 according to the present invention comprises a glass plate 15 including (1) a sample application portion 11 for applying a sample to the device 10, (2) a sample recovery portion 12 for recovering, from the device 10, the sample applied to the sample application portion 11, (3) a developing portion 13 for the sample's moving from the sample application portion 11 to the sample recovery portion 12, and (4) an antibody-carrying portion 14 for carrying an antibody capable of binding to the antigen on the developing portion 13. When an antibody sandwich method is used to detect an antigen in the sample (details are described later), the immunochromatographic device 10 may also comprise (5) a positive control portion 20 for carrying a secondary antibody capable of binding to the labeled antibody against the antigen, which is provided in the developing portion 13. One embodiment of the device 10 is shown in Fig. 1.

The glass plate 15 may comprise either a smooth or roughened surface. The developing portion 13 or the antibody-carrying portion 14 of the glass plate 15 comprises a roughened surface. The mean roughness (Ra) of the roughened glass is 0.01-20.0 µm, preferably 0.05-10.0 µm, more preferably 0.1-1 µm, still more preferably 0.2-0.3 µm, and most preferably 0.226-0.252 µm. The maximum height of roughness (Rz) of the roughened glass is 0.1-150.0 µm, preferably 0.5-100.0 µm, more preferably, 1.0-10.0 µm, still more preferably 2.0-3.0 µm, and most preferably 2.19-2.70 µm. The concave-to-convex distance (Sm) of the roughened glass is 10-2000 µm, preferably 30-1000 µm, more preferably 50-500 µm, still more preferably 100-200 µm, and most preferably 165-179 µm. The mean roughness (Ra), the maximum height (Rz), and the concave-to-convex distance (Sm) are under the definition in JIS B0601. The amount of antibodies immobilized on the antibody-carrying portion 14 can be increased by using the antibody-carrying portion 14 comprisng a roughened surface. In addition, when the antibody-carrying portion 14 comprises a roughened surface, the developing portion 13 can be manufactured more easily by making the developing portion 13 with a roughened glass.

The developing portion has a transparent plate 16. The glass plate 15 and the transparent plate 16 are placed in parallel with each other with a gap between them. The size of the gap is so determined that the sample can migrate in the developing portion by capillary action. The glass plate 15 and the transparent plate 16 may be spaced apart using, for example, a spacer. Alternatively, in the developing portion, to provide a gap between the glass plate 15 and the transparent plate 16, the surface of either plate is sunk. No spacer is required for the latter case. The distance between the glass plate 15 and the transparent plate 16 is not specifically limited as long as a sample can migrate in the developing portion by capillary action. The distance may be 1-100 µm, which is preferably 3-50 µm, and most preferably 5-30 µm. The transparent plate 16 may be any one of plates that can be used for the immunochromatographic assay. Examples include a glass plate and a plastic plate such as an acrylic plate or a polypropylene plate. As used herein, the term "transparent plate" refers not only to a plate that is transparent when dried, but also to a plate that becomes transparent during the immunochromatographic assay, for example, when the developing portion 13 is wet with buffer. Specific examples include glass or plastic comprising a roughened surface. When the transparent plate 16 is a glass plate, it may comprise either a smooth or roughened surface. The developing portion 13 does, however, comprise a roughened surface. The glass plate 15 and the transparent plate 16 may be separated from each other, or may be bonded directly or indirectly with, for example, a spacer.

The device 10 may have a spacer 17 to keep the gap between the glass plate 15 and the transparent plate 16. The thickness of the spacer 17 is equal to the distance between the glass plate 15 and the transparent plate 16. The thickness may be 1-100 µm, which is preferably 3-50 µm, and most preferably 5-30 µm. Although the spacer 17 may be made of any suitable material, it is preferable to use a material that can be worked or machined so that the spacers 17 are adhered to the glass plate 15 and the transparent plate 16. Examples of the material include glass and plastics such as acrylic resins. The spacer 17 as used herein may have any shape as long as a suitable gap can be maintained between the glass plate 15 and the transparent plate 16 in the developing portion 13. The spacer 17 may have a rectangular or cubic shape and widely contacts with the glass plate 15 and/or the transparent plate 16 two-dimentionally or linearly. The spacer 17 may have a pointed tip and contact with the glass plate 15 and/or the transparent plate 16 at a point. The number of the spacer 17 is not specifically limited and may be determined appropriately depending on their shape. The glass plate 15 and/or the transparent plate 16 may have a surface coated with a resin or an ink or printed. It is preferable that the glass plate 15, the transparent plate 16, and the spacers 17 on both sides of the developing portion 13 are adhered or in close contact with each other so that the sample flowing through the developing portion 13 does not escape from the developing portion 13. The glass plate 15, the transparent plate 16, and the spacers 17 may be adhered by using, for example, an adhesive material. Alternatively, they may be clamped tightly with a clip and the others.

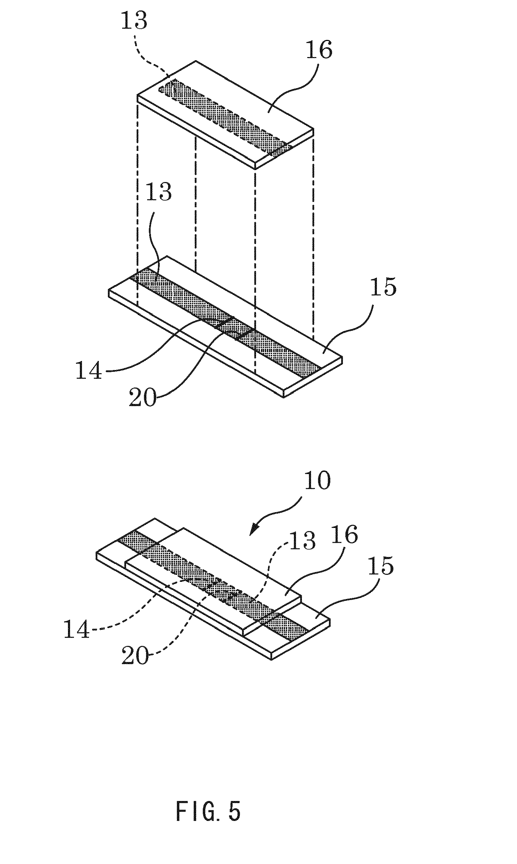

When both the glass plate 15 and the transparent plate 16 in the developing portion 13 comprise a roughened surface produced by reducing a smooth surface, the device 10 can be provided by merely putting the glass plate 15 and the transparent plate 16 together directly without any spacers as shown in Fig. 5. In other words, it becomes possible that the smooth areas of the glass plate 15 and the transparent plate 16 are made direct contact, thereby a gap, in which the sample can migrate in the developing portion 13 by capillary action, is generated in the area where the roughened surfaces are faced and the sample does not escape from the developing portion 13 due to the smooth surfaces at both sides. In this case, similar surface conditions to those described in conjunction with the glass plate 15 may be applied to the roughened surface of the transparent plate 16.

### == Immunochromatographic assay ==

Immunochromatographic assay using the aforementioned immunochromatographic device 10 may be performed in any assay format including a competitive format and a sandwich format. The sandwich assay is described below as an example.

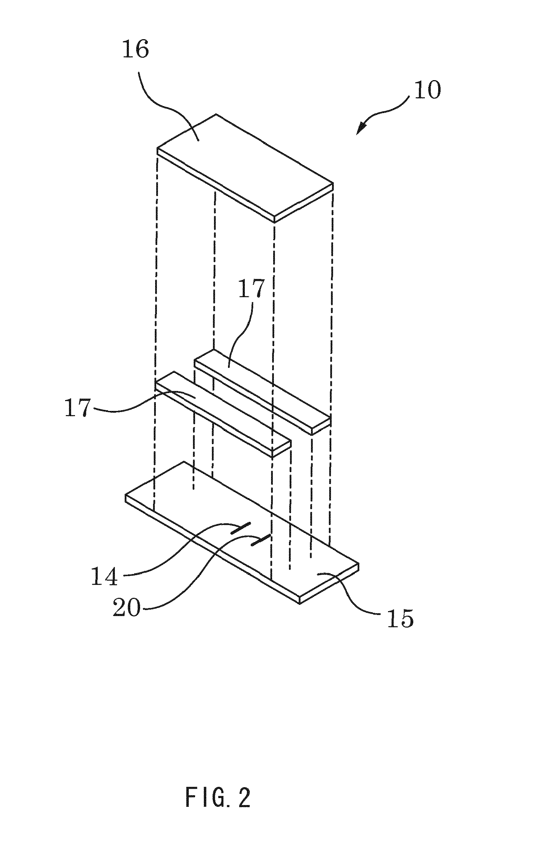

First, antibody is immobilized on the antibody-carrying portion 14 of the glass plate 15 to allow the antigen contained in the sample to directly or indirectly bind to the antibody. This may be achieved by using any one of suitable methods known to those skilled in the art, such as a silane coupling technique or a silicon-bio method (Japanese patent application No. 2006-135572). The spacers 17 and the transparent plate 16 are placed to assemble the device 10 (Fig. 2).

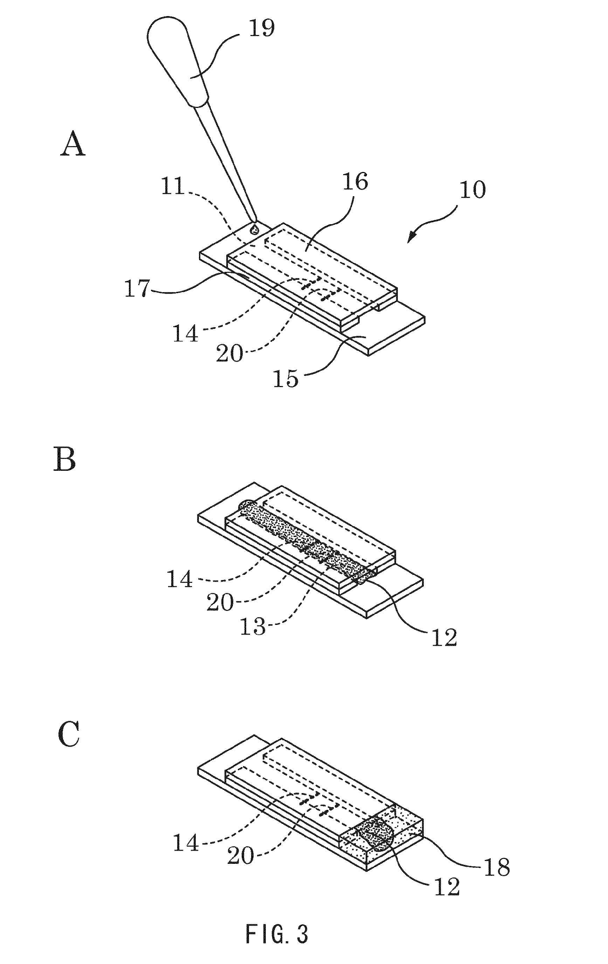

A sample containing a target antigen is mixed with a labeled antibody against the antigen. The usable label is not limited to but includes enzyme, colloidal gold, colloidal platinum-gold, and fluorescent compound. The sample containing the labeled antibody and the antigen bound to the labeled antibody is applied to the sample application portion 11 on the glass plate 15 to introduce the sample into the developing portion 13 (Fig. 3A). The sample may be introduced into the developing portion 13 using a tool 19 such as PIPETMAN (Gilson) or a micropipette or through a sample pad. The labeled antibody to the antigen may be replaced with a combination of a primary antibody that recognizes the antigen and a labeled secondary antibody that recognizes the primary antibody. The antibody immobilized on the antibody-carrying portion 14 may directly bind to the antigen or may be a secondary antibody that indirectly binds to the antigen by specifically recognizing the primary antibody that binds to the antigen. A number of their modified techniques are known in the art, all of which are included in the scope of the present invention.

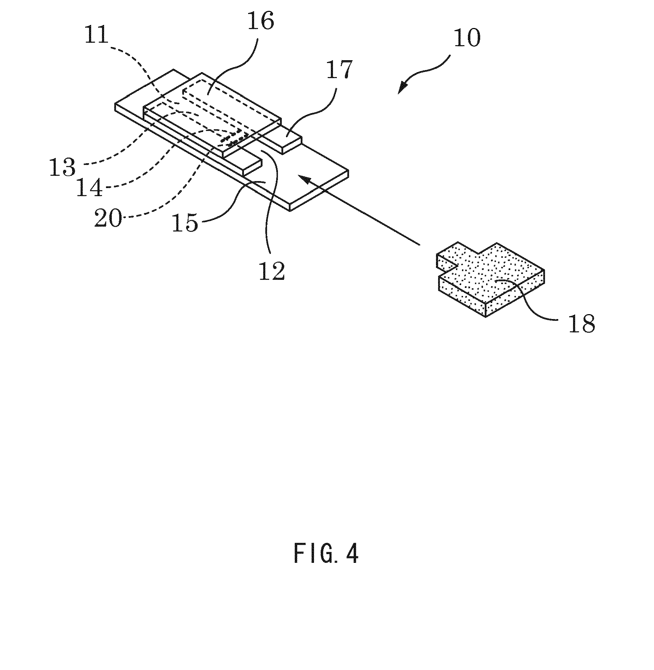

The sample introduced into the developing portion 13 spreads and migrates across the developing portion 13 by capillary action. The sample passes through the antibody-carrying portion 14 and reaches the sample recovery portion 12 (Fig. 3B). The sample is recovered from the sample recovery portion 12. The sample may be completely collected or a portion of the sample may be left in the developing portion 13. If any background signal should affect the measurement, the developing portion 13 may be washed by flushing a buffer without colloidal gold through the developing portion 13 after the flowout of the sample. How the sample is recovered is not specifically limited. It may be collected using a PIPETMAN or a micropipette. Alternatively, by putting a liquid absorber 18, such as a sample pad, a sponge, or a sheet of filter paper in the sample recovery portion 12, the sample may be absorbed into it and recovered (Figs. 3C and 4).

During this procedure, it is preferable that the antibody is immobilized in such a manner that the total volume of the sample passes through the antibody-carrying portion 14 in order to attain at a high quantitative accuracy. For example, when the developing portion 13 is like a closed channel having the sample application portion 11 and the sample recovery portion 12 at the entrance and the exit of the channel, respectively, the antibody can be immobilized along a line crossing the channel in the direction perpendicular to the channel. With this configuration, the sample flowing through the channel always passes over the immobilized antibody, taking most advantage of the antigen-binding ability of the antibody-carrying portion 14 to allow the binding of the antigen as much as possible. Similarly, when the positive control portion 20 is provided, in order to maximize its effect, it is preferable that the antibody that recognizes the labeled antibody is immobilized along a line crossing the channel in the direction perpendicular to the channel between the antibody-carrying portion 14 and the sample recovery portion 12.

Then the antigen bound to the antibody-carrying portion 14 is detected. When the labeled antibody that recognizes the antigen is used as described above, the labeled antibody captured at the antibody-carrying portion 14 can be detected using the label. For example, when enzyme-conjugated antibody is used, a substrate solution may migrate over the developing portion 13 as in the case of the sample and allowed to contact with the enzyme to develop the color. For example, luciferase used as the label emits light when provided with a substrate luciferin. A fluorescent dye such as fluorescein emits fluorescence upon excitation. When the device 10 has the positive control portion 20, the labeled antibody in the sample binds to the antibody immobilized on the positive control portion 20 and produces color or light at the positive control portion 20.

The detection of the color by the label can be performed by any one of suitable means. For example, taking advantage of the transparency of the glass plate 15 and the transparent plate 16, the transmitting light may be used to detect the color. One approach involves directing the light at an appropriate wavelength down from above and illuminating the antibody-carrying portion 14. Analysis of the amount of the transmitted light provides a quantitative measure of the signal intensity. Another approach uses a light box on which the device 10 after development of the color is placed. Digital CCD image of the device is taken and the signal intensity is calculated using a software. Alternatively, the device 10 with the colored line may be placed on a sheet of white paper to detect reflected light. It is preferable that a calibration curve is prepared each time the analysis is performed in order to provide an exact quantitative measurement; the level of the antigen, however, can be calculated directly from the signal intensity by making the conditions as constant as possible.

### Example

### (1) Preparation of an immunochromatographic chip

First, 0.5 mL of acetic acid was added to 99 mL of water while stirring, to which 0.5 mL of 3-glycidyloxypropyl-trimethoxysilane was added dropwise. The mixture was stirred for 1 hour at room temperature. Glass plates, each having a size of 5 mm x 25 mm, were immersed in this solution overnight at room temperature. The glass plates used were: glass plates with one surface roughened and a glass plate with smooth surface. Thereafter, the glass plates were washed with water and dried in the air. The glass plates were then heated for 5 minutes at 115°C to activate the surface of the glass. The features of the roughened glass plates are given in Table 1 below.

**Table 1**

| | Roughened glass I | Roughened glass II |
|---|---|---|
| Ra (mean roughness) | 0.226-0.252 µm | 14-19 µm |
| Rz (maximum height) | 2.19-2.70 µm | 95-120 µm |
| Sm (concave-to-convex distance) | 165-179 µm | 1.07-1.67 mm |

A line was drawn with 1 µL of rabbit anti-ovalbumin antibody (1 mg/mL) in 0.2 M sodium phosphate, on the roughened surface in or near the middle of the length of each glass plate. The slides were placed in a dish with a sheet of filter paper moistened with water and allowed to stand for 1 hour at room temperature to immobilize the antibody. The glass plates were washed by spraying 2 mL of TT buffer (20 mM Tris-HCl (pH 7.4) containing 0.05 % Tween 20 and 150 mM NaCl), immersed in TTB buffer (TBS/Tween containing 1 mg/mL of bovine serum albumin) and allowed to stand for 1 hour at room temperature to block any unreacted active groups.

The antibody-immobilized surface of the glass plate was adhered to a middle portion of a plastic plate having a size of 5 mm x 40 mm. The glass plate was fixed to the plastic plate with a piece of adhesive tape. The immunochromatographic chip thus obtained has a narrow space between the plastic surface and the glass surface through which liquid can flow.

Commercially available immunochromatographic sample pads (each having a size of 4 mm x 5 mm) moisturized with TTB were placed on the plastic plate at both ends of the plastic plate not covered with the glass plate, with the sample pads contacted with the glass plates. Then, 10 µL of sample was added to one sample pad. The sample used was a mixed solution containing equal amounts of ovalbumin (1, 10, 100, and 1000 ng/mL), rabbit anti-ovalbumin antibody, and colloidal gold-conjugated anti-rabbit Ig antibody. A filter paper was placed on top of the other sample pad to absorb the solution flown up through the developing portion. A total of 200 µL of sample was applied.

### (2) Analysis of the results

Images of the immunochromatographic chips after the reaction were captured by a digital camera. Subsequently, the signal intensities were calculated as Area statistics using the Analyze/Gels function (submenu) in ImageJ software.

The results are given in Table 2. The value measured at 0 ng/ml of OVA level indicates a background. When a smooth glass was used, the value measured at 1 ng/ml of OVA level was lower than the background. On the other hand, when roughened glasses were used, the value measured at 1 ng/ml of OVA level was higher than the background.

**Table 2**

| OVA level | Area | | |
|---|---|---|---|
| (ng/ml) | Smooth glass | Roughened glass I | Roughened glass II |
| 0 | 461 | 449 | 245 |
| 1 | 325 | 1217 | 395 |
| 10 | 863 | 2649 | 484 |
| 100 | 1556 | 3359 | 496 |
| 1000 | 2483 | 5711 | 921 |

As apparent from the above, the glasses comprising a roughened surface (the lowest detection level < 1 ng/ml) have higher sensitivities than the one comprising a smooth surface(the lowest detection level > 1 ng/ml). The roughened glass I has a particularly high sensitivity.

## Claims

1. An immunochromatographic device (10) comprising:
a glass plate (15) including:
a sample application portion (11) for applying a sample containing an antigen to the device;
a sample recovery portion (12) for recovering the sample applied to the sample application portion from the device;
a developing portion (13) for developing the sample from the sample application portion to the sample recovery portion; and
an antibody-carrying portion (14) for carrying an antibody capable of binding to the antigen in the developing portion; wherein
the developing portion (13) comprises a transparent plate (16),
the glass plate (15) and the transparent plate (16) are placed in parallel with each other with a gap, and
the sample can migrate in the gap of the developing portion by capillary action,
wherein the device is **characterized in that**:
the developing portion (13) or the antibody-carrying portion (14) in the glass plate (15) comprises a roughened surface, and the roughened surface has a mean roughness of 0.01-20.0 µm, a maximum height of roughness of 0.1-150.0 µm, and a concave-to-convex distance of 10-2000 µm.

2. The immunochromatographic device of Claim 1, wherein the gap has a size of 3-50 µm.

3. The immunochromatographic device of Claim 1 or 2, wherein the transparent plate (16) comprises a roughened surface at an area corresponding to the roughened surface(s) of the developing portion (13) and/or the antibody-carrying portion (14) of the glass plate (15).

4. The immunochromatographic device of Claim 5, wherein each of the developing portion (13) and the antibody-carrying portion (14) of the glass plate (15) and the transparent plate (16) comprises a roughened surface and does not comprise a spacer for keeping the gap.

5. A method for detecting an antigen in a sample using an immunochromatographic device (10),
the device comprising a glass plate (15), the glass plate (15) comprising
a sample application portion (11) for applying the sample to the device;
a sample recovery portion (12) for recovering the sample applied to the sample application portion from the device;
a developing portion (13) for developing the sample from the sample application portion to the sample recovery portion; and
an antibody-carrying portion (14) for carrying an antibody capable of binding to the antigen in the developing portion; wherein
the developing portion (13) comprises a transparent plate (16), the transparent plate (16) is placed in parallel with the glass plate (15) with a gap, and the sample can migrate in the gap of the developing portion (13) by capillary action;
the method comprising the steps of:
applying the sample to the sample application portion (11) to develop the sample through the developing portion (13) ;
recovering the sample developed through the developing portion (13) at the sample recovery portion (12); and
detecting the antigen bound to the antibody-carrying portion (14); the method **characterized in that**:
the developing portion (13) or the antibody-carrying portion (14) comprises a roughened surface and the roughened surface has a mean roughness of 0.01-20.0 µm, a maximum height of roughness of 0.1-150.0 µm, and a concave-to-convex distance of 10-2000 µm.

## Patentansprüche

1. Ein immunchromatographisches Gerät (10), das Folgendes beinhaltet:
eine Glasplatte (15), die Folgendes einschließt:
einen Probenapplikationsabschnitt (11) zum Applizieren einer Probe, die ein Antigen enthält, auf das Gerät;
einen Probenrückgewinnungsabschnitt (12) zum Rückgewinnen der auf den Probenapplikationsabschnitt applizierten Probe aus dem Gerät;
einen Entwicklungsabschnitt (13) zum Entwickeln der Probe aus dem Probenapplikationsabschnitt zu dem Probenrückgewinnungsabschnitt; und
einen antikörpertragenden Abschnitt (14) zum Tragen eines Antikörpers, der in der Lage ist, in dem Entwicklungsabschnitt an das Antigen zu binden; wobei
der Entwicklungsabschnitt (13) eine transparente Platte (16) beinhaltet,
die Glasplatte (15) und die transparente Platte (16) parallel zueinander mit einem Spalt dazwischen angeordnet sind und
die Probe durch Kapillarwirkung in dem Spalt des Entwicklungsabschnitts wandern kann,
wobei das Gerät **dadurch gekennzeichnet ist, dass**:
der Entwicklungsabschnitt (13) oder der antikörpertragende Abschnitt (14) in der Glasplatte (15) eine aufgeraute Oberfläche beinhaltet und die aufgeraute Oberfläche eine mittlere Rauheit von 0,01-20,0 µm, eine maximale Höhe der Rauheit von 0,1-150,0 µm und einen Konkav-zu-Konvex-Abstand von 10-2000 µm aufweist.

2. Das immunchromatographische Gerät gemäß Anspruch 1, wobei der Spalt eine Größe von 3-50 µm aufweist.

3. Das immunchromatographische Gerät gemäß Anspruch 1 oder 2, wobei die transparente Platte (16) eine aufgeraute Oberfläche an einer Fläche aufweist, die der (den) aufgerauten Oberfläche(n) des Entwicklungsabschnitts (13) und/oder des antikörpertragenden Abschnitts (14) der Glasplatte (15) entspricht.

4. Das immunchromatographische Gerät gemäß Anspruch 5, wobei jeder von dem Entwicklungsabschnitt (13) und dem antikörpertragenden Abschnitt (14) der Glasplatte (15) und der transparenten Platte (16) eine aufgeraute Oberfläche beinhaltet und keinen Abstandhalter beinhaltet, um den Spalt zu erhalten.

5. Ein Verfahren zum Nachweis eines Antigens in einer Probe unter Verwendung eines immunchromatographischen Geräts (10),
wobei das Gerät eine Glasplatte (15) beinhaltet, wobei die Glasplatte (15) Folgendes beinhaltet:
einen Probenapplikationsabschnitt (11) zum Applizieren der Probe auf das Gerät;
einen Probenrückgewinnungsabschnitt (12) zum Rückgewinnen der auf den Probenapplikationsabschnitt applizierten Probe aus dem Gerät;
einen Entwicklungsabschnitt (13) zum Entwickeln der Probe aus dem Probenapplikationsabschnitt zu dem Probenrückgewinnungsabschnitt; und
einen antikörpertragenden Abschnitt (14) zum Tragen eines Antikörpers, der in der Lage ist, in dem Entwicklungsabschnitt an das Antigen zu binden; wobei
der Entwicklungsabschnitt (13) eine transparente Platte (16) beinhaltet, wobei die transparente Platte (16) und die Glasplatte (15) parallel zueinander mit einem Spalt dazwischen angeordnet sind, und die Probe durch Kapillarwirkung in dem Spalt des Entwicklungsabschnitts (13) wandern kann;
wobei das Verfahren die folgenden Schritte beinhaltet:
Applizieren der Probe auf den Probenapplikationsabschnitt (11), um die Probe durch den Entwicklungsabschnitt (13) zu entwickeln;
Rückgewinnen der durch den Entwicklungsabschnitt (13) entwickelten Probe an dem Probenrückgewinnungsabschnitt (12); und
Nachweis des an den antikörpertragenden Abschnitt (14) gebundenen Antigens; wobei das Verfahren **dadurch gekennzeichnet ist, dass**:
der Entwicklungsabschnitt (13) oder der antikörpertragende Abschnitt (14) eine aufgeraute Oberfläche beinhaltet und die aufgeraute Oberfläche eine mittlere Rauheit von 0,01-20,0 µm, eine maximale Höhe der Rauheit von 0,1-150,0 µm und einen Konkav-zu-Konvex-Abstand von 10-2000 µm aufweist.

## Revendications

1. Dispositif immunochromatographique (10) comprenant :
une plaque de verre (15) comportant :
une portion d'application d'un échantillon (11) pour appliquer un échantillon contenant un antigène sur le dispositif ;
une portion de récupération de l'échantillon (12) pour récupérer du dispositif l'échantillon appliqué sur la portion d'application de l'échantillon ;
une portion de transfert (13) pour transférer l'échantillon de la portion d'application de l'échantillon à la portion de récupération de l'échantillon ; et
une portion portant un anticorps (14) pour transporter un anticorps capable de se lier à l'antigène dans la portion de transfert ; dans lequel
la portion de transfert (13) comprend une plaque transparente (16),
la plaque de verre (15) et la plaque transparente (16) sont situées parallèlement l'une par rapport à l'autre avec une brèche et l'échantillon peut migrer dans la brèche de la portion de transfert par action capillaire,
le dispositif étant **caractérisé en ce que** :
la portion de transfert (13) ou la portion portant un anticorps (14) de la plaque de verre (15) comprend une surface rugueuse et la surface rugueuse a une rugosité moyenne de 0,01-20,0 µm, une hauteur maximale des pics de rugosité de 0,1-150,0 µm et une distance concave à convexe de 10-2 000 µm.

2. Dispositif immunochromatographique de la revendication 1, dans lequel la taille de la brèche est de 3-50 µm.

3. Dispositif immunochromatographique de la revendication 1 ou 2, dans lequel la plaque transparente (16) comprend une surface rugueuse au niveau d'une zone qui concorde avec la(es) surface(s) rugueuse(s) de la portion de transfert (13) et/ou de la portion portant un anticorps (14) de la plaque de verre (15).

4. Dispositif immunochromatographique de la revendication 5, dans lequel la portion de transfert (13) et la portion portant un anticorps (14) de la plaque de verre (15) et la plaque transparente (16) comprennent chacune une surface rugueuse et ne comportent pas un espaceur pour maintenir la brèche.

5. Procédé de détection d'un antigène dans un échantillon au moyen d'un dispositif immunochromatographique (10),
le dispositif comprenant une plaque de verre (15) et la plaque de verre (15) comportant
une portion d'application d'un échantillon (11) pour appliquer un échantillon sur le dispositif ;
une portion de récupération de l'échantillon (12) pour récupérer du dispositif l'échantillon appliqué sur la portion d'application de l'échantillon ;
une portion de transfert (13) pour transférer l'échantillon de la portion d'application de l'échantillon à la portion de récupération de l'échantillon ; et
une portion portant un anticorps (14) pour transporter un anticorps capable de se lier à l'antigène dans la portion de transfert ; dans lequel
la portion de transfert (13) comprend une plaque transparente (16), la plaque transparente (16) est située parallèlement à la plaque de verre (15) avec une brèche et l'échantillon peut migrer dans la brèche de la portion de transfert (13) par action capillaire ;
le procédé comprenant les étapes qui consistent à :
appliquer l'échantillon dans la portion d'application de l'échantillon (11) pour transférer l'échantillon au travers de la portion de transfert (13) ;
récupérer l'échantillon développé au travers de la portion de transfert (13) dans la portion de récupération de l'échantillon (12) ; et
détecter l'antigène lié à la portion portant un anticorps (14) ; le procédé étant **caractérisé en ce que** :
la portion de transfert (13) ou la portion portant un anticorps (14) comprend une surface rugueuse et la surface rugueuse a une rugosité moyenne de 0,01-20,0 µm, une hauteur maximale des pics de rugosité de 0,1-150.0 µm et une distance concave à convexe de 10-2 000 µm.
